# EUROPEAN PATENT APPLICATION

(11) **EP 4 699 569 A1**
(43) Date of publication of application: **25.02.2026**
(21) Application number: 25193835.3
(22) Date of filing: 04.08.2025
(51) Int. Cl.: A61F 2/07

(54) **AORTIC IMPLANTS WITH PLASMA MODIFIED SURFACE CHARACTERISTICS**

(30) Priority: 12.08.2024 US 202463682124 P; 31.07.2025 US 202519286364
(71) Applicant: Medtronic Vascular Inc., Santa Rosa, CA 95403 (US)
(72) Inventor: Van Helvoirt, Job, Santa Rosa (US); Bayon, Yves, Santa Rosa (US); Overeem, Simon, Santa Rosa (US); Lefranc, Olivier, Santa Rosa (US); Simons, Damien, Santa Rosa (US); Perkins, Keith, Santa Rosa (US)
(74) Representative: Zimmermann & Partner Patentanwälte mbB

(57) **Abstract**

An endovascular implant including a tubular body. The tubular body extends along a longitudinal axis and is bounded by a proximal end and a distal end. The tubular body has an outer surface and an inner surface. The tubular body has an outer surface region including the outer surface and an inner surface region including the inner surface and a bulk region extending therebetween. The tubular body includes an outer surface landing region within the outer surface region and proximate at least one of the proximal and distal ends of the tubular body. The outer surface landing region is configured to align with one or more landing walls of one or more vessels. The outer surface landing region includes a plasma modified outer surface landing region having a different surface compared to an unmodified outer surface landing region.

## Description

### CROSS-REFERENCE TO RELATED APPLICATIONS

This application claims the benefit of U.S. Provisional Patent Application Serial No. 63/682,124, filed August 12, 2024, and U.S. Patent Application Serial No. 19/286,364, filed July 31, 2025, the entire contents of which are incorporated herein by reference.

### TECHNICAL FIELD

The present disclosure relates to aortic implants with plasma modified surface characteristics. The aortic implant may be an endovascular stent graft. The plasma modified surface characteristics may be imparted on a surface portion of the endovascular stent graft.

### BACKGROUND

An aortic aneurysm may be an enlarged area in an aorta (e.g., an abdominal aorta). The aortic aneurysm may form from the degradation of elastin and/or interstitial collagen, which may alter the structural integrity of the aortic wall, which may weaken it. One current treatment of an aortic aneurysm may be endovascular surgery which utilizes an implant (e.g., stent graft). Another more invasive option is open surgery.

Endovascular procedures are minimally invasive techniques to deliver clinical treatments in a patient's vasculature (e.g., treatment of aortic aneurysms). One example of a clinical treatment used in an endovascular procedure is deployment of a stent graft. A conventional stent graft typically includes a radially expandable reinforcement structure, e.g., formed from a plurality of annular stent rings, and a cylindrically shaped layer of graft material defining a lumen to which the stent rings are coupled. The stent graft is placed inside a patient's vasculature (e.g., blood vessel) to bridge a diseased blood vessel segment (e.g., an aneurismal, dissected, or torn blood vessel segment), and thereby excluding hemodynamic pressures of blood flow from the diseased blood vessel segment.

### SUMMARY

One embodiment includes an endovascular implant including a tubular body. The tubular body extends along a longitudinal axis and is bounded by a proximal end and a distal end. The tubular body has an outer surface and an inner surface. The tubular body has an outer surface region including the outer surface and an inner surface region including the inner surface and a bulk region extending therebetween. The tubular body includes an outer surface landing region within the outer surface region and proximate at least one of the proximal and distal ends of the tubular body. The outer surface landing region is configured to align with one or more landing walls of one or more vessels. The outer surface landing region includes a plasma modified outer surface landing region having a different surface compared to an unmodified outer surface landing region.

The plasma modified outer surface landing region may be configured to promote cellular adhesion and/or cellular ingrowth. The plasma modified outer surface landing region may have one or more modified characteristics and the unmodified outer surface landing region may have one or more unmodified characteristics. The one or more modified characteristics may include a modified surface roughness and the one or more unmodified characteristics may include an unmodified surface roughness. The modified surface roughness may be greater than the unmodified surface roughness. The plasma modified outer surface landing region may include covalently bonded molecules imparted from feed molecules of a plasma treatment. The feed molecules may include one or more amine molecules, one or more molecules having hydroxyl groups, one or more molecules having carboxyl groups, or a combination thereof. The feed molecules may be one or more biologic molecules. The biologic molecules may be selected from the group consisting of collagen, hyaluronic acid, fibronectin, amine molecules, microRNA, antimicrobial peptides, or a combination thereof.

The plasma modified outer surface landing region may include alternating first and second modified regions formed using first and second plasma treatments. The second plasma treatment may be different than the first plasma treatment. The first modified regions may have a first surface roughness. The second modified surface region may have a second surface roughness. The first surface roughness may be different than the second surface roughness.

In another embodiment an endovascular implant is disclosed. The endovascular implant includes a tubular body extending along a longitudinal axis and bounded by a proximal end and a distal end. the tubular body has an outer surface and an inner surface. The tubular body has an outer surface region including the outer surface and an inner surface region including the inner surface and a bulk region extending therebetween. The inner surface region is configured to contact blood in a deployed state of the endovascular implant. The inner surface region includes a plasma modified inner surface region having a different inner surface compared to an unmodified inner surface region.

The plasma modified inner surface region may be configured to decrease thrombogenicity from the blood. The plasma modified inner surface region may have one or more modified characteristics. The unmodified inner surface region may have one or more unmodified characteristics. The one or more modified characteristics may include a modified surface smoothness. The one or more unmodified characteristics may include an unmodified surface smoothness. The modified surface smoothness may be greater than the unmodified surface smoothness. The plasma modified inner surface region may include covalently bonded molecules imparted from feed molecules of a plasma treatment. The feed molecules may be heparin, albumin, polyethylene glycol (PEG), antithrombin, endothelial cell-derived molecules, and/or combinations thereof. The feed molecules may be acrylates, siloxanes, and/or vinyl groups containing precursors with amino, hydroxyl, carboxyl and/or epoxy functionalities.

In yet another embodiment an endovascular implant is disclosed. The endovascular implant includes a tubular body extending along a longitudinal axis and bounded by a proximal end and a distal end. The tubular body has an outer surface and an inner surface. The tubular body has an outer surface region including the outer surface and an inner surface region including the inner surface and a bulk region extending therebetween. The outer surface region includes a plasma modified outer surface region having a different outer surface compared to an unmodified outer surface region.

The plasma modified outer surface region may be configured to increase thrombogenicity and/or to promote immune system modulation. The plasma modified outer surface region may have one or more modified characteristics and the unmodified outer surface region may have one or more unmodified characteristics. The one or more modified characteristics may include a modified surface roughness. The one or more unmodified characteristics may include an unmodified surface roughness. The modified surface roughness may be greater than the unmodified surface roughness. The plasma modified outer surface landing region may include covalently bonded molecules imparted from feed molecules of the plasma treatment. The feed molecules may include one or more polydimethylsiloxane (PDMS), siloxanes, fluorinated, long chain hydrocarbons, or a combination thereof.

A method of plasma treating an endovascular implant is disclosed. The method includes providing a tubular body extending along a longitudinal axis and bounded by a proximal end and a distal end. The tubular body has an outer surface and an inner surface. The method further includes the step of treating at least a portion of the outer surface and/or the inner surface with plasma to obtain a modified surface region having one or more modified surface characteristics. The at least a portion of the outer surface and/or the inner surface has one or more unmodified surface characteristics.

In one or more method embodiments, the at least a portion of the outer surface and/or the inner surface is an outer surface landing region aligning with one or more landing walls of one or more vessels when the tubular body is in a deployed position and the modified surface region is a modified outer surface landing region. The one or more modified surface characteristics may be a modified surface roughness and the one or more unmodified characteristics may include an unmodified surface roughness. The modified surface roughness may be greater than the unmodified surface roughness. The modified outer surface landing region may include covalently bonded molecules imparted from feed molecules of a plasma treatment.

In one or more method embodiments, the modified outer surface landing region includes alternating first and second modified regions formed using first and second plasma treatments, the second plasma treatment is different than the first plasma treatment, the first modified regions have a first surface roughness, the second modified surface regions have a second surface roughness, and the first surface roughness is different than the second surface roughness.

In one or more method embodiments, the at least a portion of the outer surface and/or the inner surface is an inner surface region and the modified surface region is a modified inner surface region. The one or more modified surface characteristics may be a modified surface smoothness and the one or more unmodified characteristics may include an unmodified surface smoothness. The modified surface smoothness may be greater than the unmodified surface roughness. The modified inner surface region may include covalently bonded molecules imparted from feed molecules of a plasma treatment.

In one or more method embodiments, the at least a portion of the outer surface and/or the inner surface is an outer surface region and the modified surface region is a modified outer surface region. The one or more modified surface characteristics may be a modified surface roughness and the one or more unmodified characteristics may include an unmodified surface roughness. The modified surface roughness may be greater than the unmodified surface roughness. The modified outer surface region may include covalently bonded molecules imparted from feed molecules of a plasma treatment. The at least a portion of the outer surface and/or the inner surface may be an inner surface region and an outer surface region.

### BRIEF DESCRIPTION OF THE DRAWINGS

Figure 1 is a partial cross section view of an abdominal aorta depicting a side view of a stent graft extending within the abdominal aorta and a first common iliac artery (CIA) and a second CIA.
Figure 2 is a partial cross section view of the abdominal aorta of Figure 1 and a side view of the stent graft, the first branch limb extension, and the second branch limb extension of Figure 1 with modified outer surface portions to promote cellular adhesion and/or cellular ingrowth.
Figure 3 is a schematic view of a modified outer surface portion having alternating first and second outer surface portions having different modified surface morphologies.
Figure 4 is a partial cross section view of the abdominal aorta of Figure 1 and a side view of the stent graft, the first branch limb extension, and the second branch limb extension of Figure 1 with a modified inner surface portion to decrease thrombogenicity.
Figure 5 is a partial cross section view of the abdominal aorta of Figure 1 and a side view of the stent graft, the first branch limb extension, and the second branch limb extension of Figure 1 with a modified outer surface portion to increase thrombogenicity and/or to promote immune system modulation.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful.

Endovascular stent grafts may be used in the treatment of abdominal aortic aneurysms. The stent graft may include straight and bifurcated portions and may be fabricated from a woven textile material or a non-woven textile material (e.g., an expanded polymer such as expanded polytetrafluoroethylene (ePTFE)) supported by a stent (e.g., a stent formed of nitinol). Physicians may use endovascular stent grafts for endovascular aneurysm repair (EVAR). Stent grafts may also be utilized during open surgery to treat abdominal aortic aneurysms (AAAs), thoracic aortic aneurysms (TAAs), or aneurysms in other locations.

The use of current endovascular stent grafts in the aorta may be susceptible to one or more possible complications that may potentially decrease the likelihood of therapy success. For instance, once positioned at a deployment site, a stent graft may be anchored on a proximal healthy segment of an aortic vessel with barbs (e.g., an anchoring stent) to minimize migration of the stent graft from the deployment site and promote sealing of the stent graft against the aortic wall at the deployment site. If the sealing of the stent graft is compromised, a Type 1 endoleak may lead to re-pressurization of an aortic aneurysm sac, thereby risking expansion of the aneurysm sac and rupture of the aortic vessel. The seal may also be compromised if the aneurysm or diseased area progresses or worsens in the direction of the seal zone.

Stent grafts are configured to ensure adequate blood flow through the aorta. However, limb graft occlusions may occur after an EVAR procedure, thereby potentially compromising blood flow and the effectiveness of the procedure. A limb graft occlusion happens when the blood flow through the graft limb becomes obstructed, which may lead to ischemia in the regions supplied by the affected vessel. This condition may result in limb pain, decreased mobility, and/or limb loss. Limb graft occlusion may depend on the structure of the stent graft and/or the material used for the stent graft.

As another example of a complication, thrombogenicity of the material (e.g., the propensity to form a blood clot) used in the stent graft may significantly influence the occurrence of Type 2 endoleaks after an EVAR procedure. Type 2 endoleaks may happen when blood continues to flow into an aneurysmal sac via collateral lumbar arteries, despite the main blood flow being diverted through the stent graft. If the material of the stent graft has a high thrombogenicity, it can promote blood clot formation with the aneurysmal sac, potentially embolizing the lumbar arteries, thereby minimizing or eliminating blood flow into the aneurysmal sac. Type 2 endoleaks are closely correlated with sac regression or expansion. If an aneurysmal sac continues to fill with blood due to an endoleak, it may not regress and it may expand, increasing the risk of rupture. Effective management of the thrombogenicity on the outside of the stent graft may help promote therapy success. While thrombogenicity may be beneficial on the outside surface of the stent graft to reduce Type 2 endoleaks, thrombogenicity on the inside (luminal) surface may lead to occlusions or other negative effects.

Yet another example of a potential complication is graft infection from bacteria adhering to the graft material. The surface characteristics of the stent graft may contribute to adherence of bacteria to the graft material. Surface characteristics that promote proper cell colonization may also protect the stent graft from bacteria clinging to the graft material.

In light of the foregoing, what is needed is a stent graft and a method for treating a stent graft to promote therapy success by reducing complications (e.g., endoleaks and/or limb graft occlusion) and/or promoting thrombogenicity and/or proper cell colonization in desired regions. In one or more embodiments, a method is disclosed for achieving one or more of these benefits. The method may include plasma treating at least portions of the outer surface and/or inner surface of the stent graft (e.g., without modifying the characteristics of the bulk region). Treatment of the outer surface may include treatment of the graft fabric (e.g., nonwoven or woven graft fabric) and/or the surfaces of one or more of the stents. The plasma may be vacuum or atmospheric. Possible treatments include chemical vapor deposition (CVD) or plasma-enhanced chemical vapor deposition (PECVD). The plasma treatment may also be a pre-incubation of the stent with blood plasma. The depth of the surface portion treated may be any of the following values or in a range of any two of the following values: 0.5, 0.6, 0.7, 0.8, 0.9, 1.0, 1.1, 1.2, 1.3, 1.4, 1.5, 1.6, 1.7, 1.8, 1.9, 2.0, 5.0, 10.0, 15.0, 20.0, 25.0, 30.0, 35.0, 40.0, 45.0, and 50.0 nanometers. Plasma treatment of the stent graft uses plasma (e.g., partially ionized gas) to alter one or more surface properties of the outer surface and/or inner surface of the stent graft. The plasma treatment may not affect the bulk properties of the stent graft material. The partially ionized gas may be used to expose a stent graft surface to energetic ions and electrons to clean and to mechanically modify (e.g., form etching) on the stent graft surface. The plasma treatment may also include treating the stent graft surface with chemistry to create functional groups bonded (e.g., covalently bonded) to the stent graft surface.

The modified surface portion may modify one or more surface characteristics (e.g., roughness, wettability, hydrophilicity, surface chemistry, etc.), thereby modifying how a stent graft interacts with its environment when deployed within an aorta. For instance, enhancing surface hydrophilicity through plasma treatment may improve cell adhesion and proliferation. Additionally, introducing specific functional groups to the modified surface of a stent graft, for example, by molecular plasma treatment, may promote or inhibit protein adsorption, thereby controlling cellular behaviors such as adhesion, differentiation, and/or migration. As a non-limiting example, hydroxyethyl methacrylate (HEMA) may be grafted covalently at a surface of a stent graft and further polymerized into p(HEMA) to lower thrombogenicity at the surface. Other non-limiting functional groups that may be used include acrylate derivatives and vinyl esters. The modified surface portion may permit control of biological responses in connection with the use of implants (e.g., stent grafts).

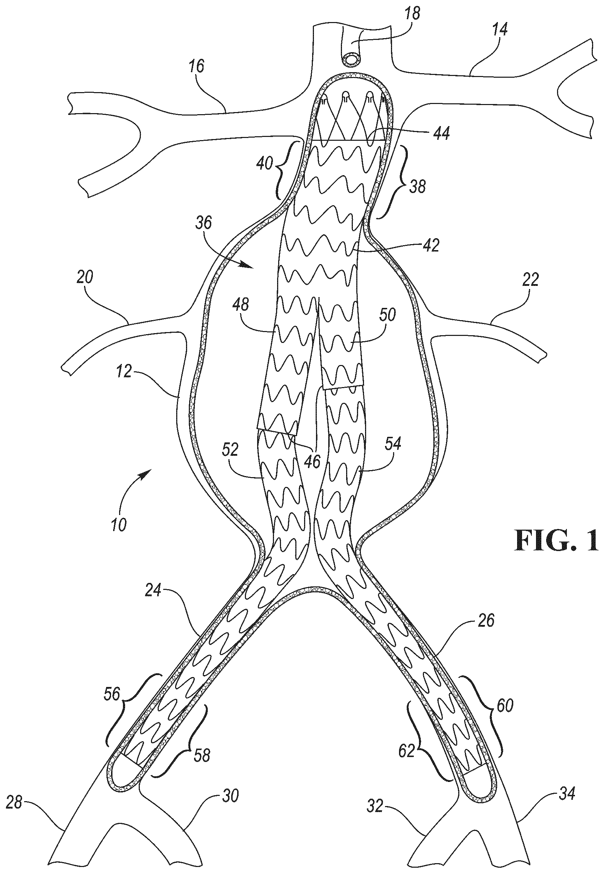

Figure 1 is a partial cross section view of abdominal aorta 10 with aneurysm 12 in the infrarenal region of abdominal aorta 10. A proximal region of abdominal aorta 10 branches into left renal artery 14, right renal artery 16, and superior mesenteric artery (SMA) 18. A medial region of abdominal aorta 10 branches into first lumbar artery 20 and second lumbar artery 22. A distal region of abdominal aorta 10 branches into first common iliac artery (CIA) 24 and second CIA 26. First CIA 24 branches into first external iliac artery (EIA) 28 and first internal iliac artery (IIA) 30. Second CIA 26 branches into second EIA 34 and second IIA 32.

Figure 1 also depicts a side view of stent graft 36 shown in an expanded, deployed configuration where proximal region 38 aligns with landing wall or zone 40 of abdominal aorta 10. Landing wall 40 extends between left renal artery 14 and right renal artery 16 and aneurysm 12. Stent graft 36 includes stent graft body 42 extending between proximal end 44 and distal end 46. Stent graft 36 includes first branch limb 48 and second branch limb 50. First branch limb extension 52 extends from first branch limb 48 into first CIA 24. Second branch limb extension 54 extends from second branch limb 50 into second CIA 26. First distal region 56 of first branch limb extension 52 aligns with landing wall or zone 58 of first CIA 24. Second distal region 60 of second branch limb extension 54 aligns with landing wall or zone 62 of second CIA 26.

While Figure 1 depicts a three-piece stent graft configuration, one or more embodiments may be applied to other configurations such as aortic extensions as well as twopiece configurations. There are also abdominal, arch and iliac branch configurations (e.g., fenestrated or branched) within one or more embodiments. One or more embodiments may also apply to other anatomical locations such as the ascending aorta, arch and/or descending thoracic aorta and to configurations for treating these anatomies. Other pathologies may be treated with one or more embodiments. Other pathologies may include dissections, transections, or a penetrating atherosclerotic ulcer (PAU).

Complications may occur after deployment of a stent graft that may reduce the likelihood of success of the stent graft therapy. A Type 1a endoleak may occur at the interface between proximal region 38 of stent graft 36 and landing wall 40. A Type 1a endoleak is a leakage of blood from abdominal aorta 10 into aneurysm 12 between the outside surface of proximal region 38 of stent graft 36 and landing wall 40. The Type 1a endoleak may lead to expansion and rupture of the aneurysmal sac. A Type 2 endoleak may occur within the sac formed by aneurysm 12. A Type 2 endoleak is the leakage of blood into the sac of aneurysm 12 from first lumbar artery 20 and/or second lumbar artery 22. A Type 2 endoleak may lead to expansion and rupture of the aneurysmal sac. Limb graft occlusion may occur within first branch limb extension 52 and/or second branch limb extension 54. A limb graft occlusion may cause a thrombotic obstruction on the inside surface (e.g., the luminal side) of a stent graft (e.g., first branch limb extension 52 and/or second branch limb extension 54). The limb graft occlusion may result in limb pain, decreased mobility, and in severe cases, limb loss. In certain anatomical locations, a limb graft occlusion may cause organ failure, stroke or even death. This may be especially problematic in the aortic arch where endovascular repair is adversely impacted by high stroke risks associated with treatment modality. A Type 1b endoleak may occur at the interface between first distal region 56 of first branch limb extension 52 and landing wall 58 of first CIA 24, or the interface between second distal region 60 of second branch limb extension 54 and landing wall 60 of second CIA 26. A Type 1b endoleak is a leakage of blood from abdominal aorta 10 into aneurysm 12 between the outside surface of first distal region 56 of first branch limb extension 52 and landing wall 58 or second distal region 60 of second branch limb extension 54 and landing wall 62. A Type 1b endoleak may lead to expansion and rupture of the aneurysmal sac. One or more embodiments are used to reduce the occurrences of one or more of the complications disclosed herein.

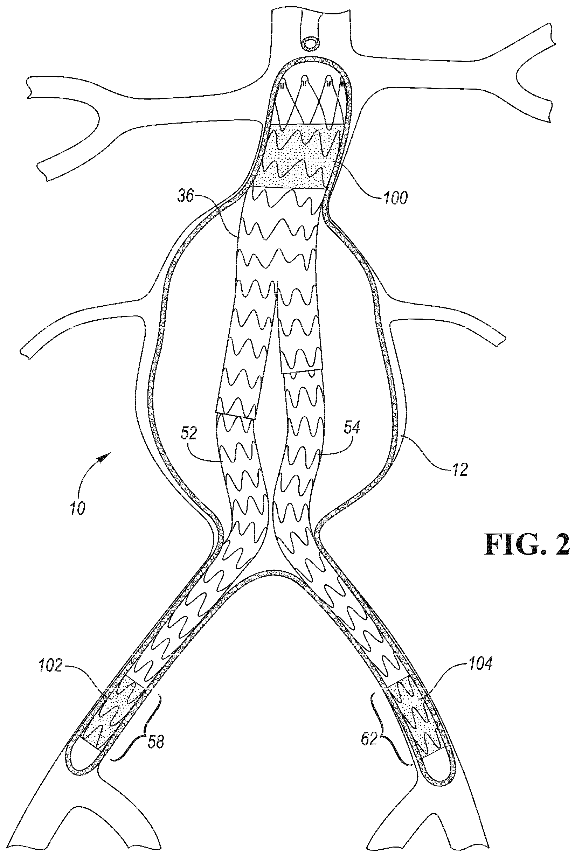

Figure 2 is a partial cross section view of abdominal aorta 10 with aneurysm 12 and a side view of stent graft 36, first branch limb extension 52, and second branch limb extension 54 shown in an expanded, deployed configuration and depicting modified outer surface portions. As shown in Figure 2, the modified outer surface portions include proximal modified outer surface portion 100, first distal modified outer surface portion 102, and second distal modified outer surface portion 104. Although Figure 2 depicts three modified outer surface portions, stent graft 36, first branch limb extension 52, and second branch limb extension 54 may include less than three modified outer surface portions or only one modified outer surface portion. For instance, stent graft 36 may include proximal modified outer surface portion 100, and first distal modified outer surface portion 102 and second distal modified outer surface portion 104 are not included on first branch limb extension 52 and second branch limb extension 54, respectively.

Proximal modified outer surface portion 100 aligns with landing wall 40 to form a proximal sealing region for stent graft 36. The length of proximal modified outer surface portion 100 may be any of the following lengths or in a range of any two of the following lengths: 10, 11, 12, 13, 14, 15, 16, 17, 18, 19, and 20 millimeters. In other embodiments, the length may be from 1 to 5 centimeters. The length of proximal modified outer surface portion 100 may extend over the entire or substantially entire proximal sealing region. In other embodiments, the portion 100 may extend over a majority of the proximal sealing region, either by length and/or by area. In further embodiments, the portion 100 may extend over at least 75% of the proximal sealing region, either by length and/or by area. First distal modified outer surface portion 102 aligns with landing wall 58 of first CIA 24 to form a first distal sealing region for first branch limb extension 52. The length of first distal modified outer surface portion 102 may be any of the following lengths or in a range of any two of the following lengths: 10, 11, 12, 13, 14, 15, 16, 17, 18, 19, and 20 millimeters. In other embodiments, the length may be greater than or equal to 15 millimeters. Second distal modified outer surface portion 104 aligns with landing wall 62 of second CIA 26 to form a second distal sealing region for second branch limb extension 54. The length of second distal modified outer surface portion 104 may be any of the following lengths or in a range of any two of the following lengths: 10, 11, 12, 13, 14, 15, 16, 17, 18, 19, and 20 millimeters. In other embodiments, the length may be greater than or equal to 15 millimeters. The length of the first and/or second distal modified outer surface portions 102, 104 may extend over the entire or substantially entire distal sealing regions. In other embodiments, the portion 102 and/or 104 may extend over a majority of the distal sealing regions, either by length and/or by area. In further embodiments, the portions 102 and/or 104 may extend over at least 75% of the distal sealing regions, either by length and/or by area.

A modified outer surface portion may be formed by treating an unmodified outer surface portion with a plasma material. In one or more embodiments, the modified outer surface portion is configured to promote strong cellular adhesion and/or cellular ingrowth to resist or prevent migration of the stent graft having the modified outer surface portion, thereby resisting or preventing endoleaks (e.g., Type 1c and/or Type 1b endoleaks). Cellular adhesion may be qualified by in vitro immunostaining to qualify single cell adhesion and quality. Additionally, force measurements may be performed to quantify adhesion force. In vivo histology may quantify cellular attachment to the material and tissue quality. Cellular ingrowth may be quantified by penetration depth. Cellular adhesion and/or cellular ingrowth may be measured as a percentage with 0% signifying no cells on the surface and thus no force or ingrowth and 100% signifying full coverage of cells or ingrowth. The cellular adhesion and/or cellular ingrowth may be 20%, 30%, 40%, 50%, 60%, 70%, 80%, 90%, and 100%. The modified outer surface portion may have one or more outer surface portion characteristics (e.g., surface roughness, surface relief, wettability, hydrophilicity, and/or stiffness) to promote strong cellular adhesion and/or cellular ingrowth. The surface roughness of one or more embodiments may be any of the following values or in a range of any two of the following values: 5, 6, 7, 8, 9, 10, 11, 12, 13, 14, 15, 20, 25, 30, 35, 40, 45, and 50 nanometers, to promote beneficial cell adhesion and/or proliferation. The wettability and/or hydrophilicity may be measured by water contact angle. The water contact angle of one or more embodiments may be any of the following values or in a range of any two of the following values: 30°, 35°, 40°, 45°, 50°, 55°, 60°, 65°, 70°, 75°, 80°, 85°, and 90° to promote beneficial cell adhesion and/or proliferation. The stiffness to promote beneficial cell adhesions and/or proliferation may be any of the following values or in a range of any two of the following values: 20, 25, 30, 35, 40, 45, 50, 55, 60, 65, 70, 75, 80, 85, 90, 95, and 100 kPa. The one or more surface portion characteristics may be imparted to the modified outer surface portion using one or more plasma treatment parameters (e.g., feed gas, power applied, power flux, treatment duration, and/or feed molecules). Non-limiting examples of feed gases/molecules include oxygen, argon, nitrogen, allylamine, aminopropyl silane, heparin, aminated-siloxanes, and combinations thereof. Nitrogen may be used for activation. Power, flux, and/or duration may be adapted to the treatment conditions (e.g., type of plasma and/or treatment region size). The plasma may be vacuum or atmospheric. Possible treatments include chemical vapor deposition (CVD) or plasma-enhanced chemical vapor deposition (PECVD).

The plasma treatment may form a mechanically modified outer surface portion to promote strong cellular adhesion and/or cellular ingrowth. The mechanically modified outer surface portion may have beneficial surface roughness and/or surface relief to promote strong cellular adhesion and/or cellular ingrowth. The surface roughness may be relatively deep so that cells can infiltrate into the pits formed by the treatment. These pits may be on a macro scale. The surface roughness may alternatively or additionally include a nanoscale roughness with an average roughness (Ra) that can be increased to get more surface of the cells for adhesion. A relatively high energy treatment and/or relatively long treatment duration may be used to increase surface roughness and/or surface relief. The treatment energy may vary depending on the machine used. Depending on the machine and the implementation, a relatively low range of 10 to 100 W, a relatively intermediate range of 100 to 500 W, or a relatively high range of 500 W or greater may be used. The treatment duration may depend on the type of plasma and/or treatment. Depending on one or more of these factors, a relatively fast range of 1 to 10 seconds, a relatively intermediate range of 1 to 10 minutes, and a relatively long range of 1 to 3 hours may be used. The surface roughness of the modified outer surface portion may be higher than the surface roughness of the unmodified outer surface portion. The surface relief of the modified outer surface portion may be higher than the unmodified outer surface portion. The surface roughness and/or relief may be modified at a microscale. The plasma treatment may impact the filaments (e.g., about 10 µm) composing the threads of woven graft material. The surface relief and/or roughness may include structures (e.g., microscale structure) such as ridges, grooves, holes, fibers, random points, pillars, bumps, nodules, honeycomb patters, cones, and combinations thereof.

The plasma treatment may form a molecular modified outer surface portion to promote strong cellular adhesion and/or cellular ingrowth. The molecular modified outer surface portion may include molecules that are covalently bonded to the outer surface portion. The plasma treatment may include treating the outer surface portion with molecules to form moieties on the outer surface portion to promote strong cellular adhesion and/or cellular ingrowth. Non-limiting examples of feed molecules for this purpose include amine molecules, molecules having hydroxyl groups, and molecules having carboxyl groups. The plasma treatment may include treating the outer surface portion with molecules (e.g., biologic molecules) to form moieties on the outer surface portion to enhance cellular attachment of endothelial cells and/or smooth muscle cells). Non-limiting examples of feed molecules for this purpose include collagen, hyaluronic acid, fibronectin, amine molecules, microRNA, and antimicrobial peptides. The molecules added by the plasma treatment may not be present on the surface(s) of the graft material that were not plasma treated.

The plasma treatment may form a functional modified outer surface portion to promote strong cellular adhesion and/or cellular ingrowth (e.g., enhance cellular attachment of endothelial cells and/or smooth muscle cells). For instance, the function of the outer surface may be modified to increase the wettability and/or hydrophilicity of the outer surface portion. The wettability and/or hydrophilicity may be any of the following values or in a range of any two of the following values: 30°, 35°, 40°, 45°, 50°, 55°, 60°, 65°, 70°, 75°, 80°, 85°, and 90°. For example, using an oxygen feed gas to the plasma treatment may increase the hydrophilicity of the outer surface portion. Increased duration of the plasma treatment may increase wettability of the outer surface portion.

The modified outer surface portion may include one or more (e.g., any or all) of the modifications (e.g., mechanical, molecular, and functional modifications).

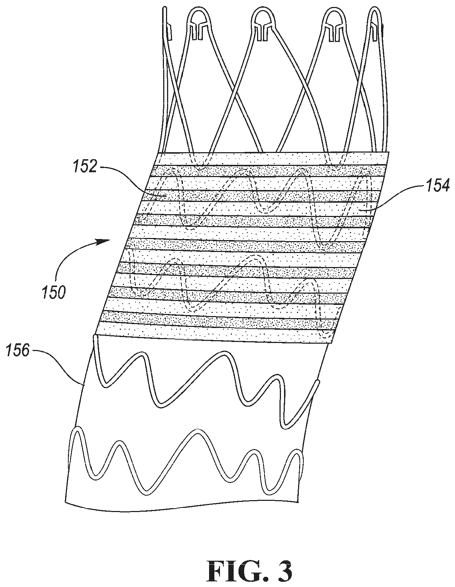

Figure 3 is a schematic view of modified outer surface portion 150 having alternating first and second modified outer surface portions 152 and 154. Alternating first and second modified outer surface portions 152 and 154 circumferentially extend around a portion of stent graft 156 (e.g., forming bands). As shown in Figure 3, alternating first and second modified outer surface portions 152 and 154 have the same length along stent graft 156. In other embodiments, the length of each of the alternating first and second modified outer surface portions 152 and 154 may be varied. In one or more embodiments, first modified outer surface portion 152 has a first modified outer surface including a first roughness and second modified outer surface portion 154 has a second modified outer surface including a second roughness. The first modified outer surface may be formed using a first plasma treatment. The second modified outer surface may be formed using a second plasma treatment. The first plasma treatment may have one or more plasma treatment parameters differing from the one or more plasma treatment parameters of the second plasma treatment. The first surface roughness may be higher than the second surface roughness, where both modified surface roughnesses are higher than the unmodified surface roughness. This differential surface roughness between the first and second modified surface portions may promote cellular adhesion and/or cellular ingrowth to resist or prevent migration of stent graft 156 and/or endoleaks (e.g., Type 1a and/or Type 1b endoleaks). In other embodiments, there may be unmodified surface portions between the modified surface portions or there may be only one kind of modified surface portion that alternates with an unmodified surface portion (e.g., an unmodified portion replaces second modified surface portion). While the portions are shown as alternating, in other embodiments the alternating is not uniform or there may be a single first modified portion and a single second modified portion (e.g., axially spaced). While the portions are shown as axially spaced bands, in other embodiments, the modified portions may be vertical stripes or bands that alternate in the circumferential direction. The alternating pattern (axially or circumferentially) illustrated in Fig. 3 may be applied to any portion disclosed herein as being treated or modified (e.g., any of the portions shown or described with respect to Figs. 2, 4, or 5) and that any portion referred to as modified or treated could be modified or treated as shown or described with respect to Fig. 3.

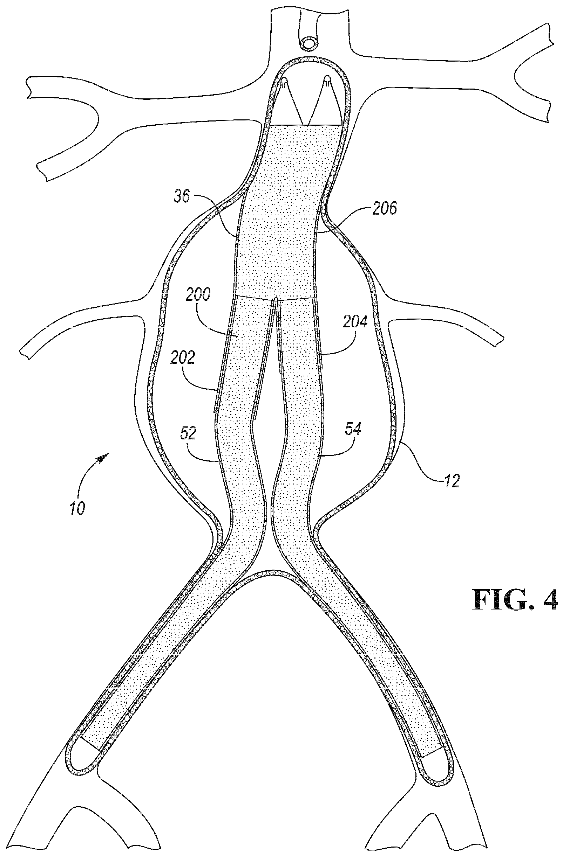

Figure 4 is a partial cross section view of abdominal aorta 10 with aneurysm 12 and a side view of stent graft 36, first branch limb extension 52, and second branch limb extension 54 shown in an expanded, deployed configuration and depicting modified inner surface portion 200. As shown in Figure 4, modified inner surface portion 200 includes all exposed inner surface portions of stent graft 36, first branch limb extension 52, and second branch limb extension 54. In other embodiments, modified inner surface portion 200 may only extend to selected portions of the exposed inner surfaces. For example, modified inner surface portion 200 may include at least a portion of the inner surface of stent graft 36. As another example, modified inner surface portion 200 may include at least a portion of the inner surface of first and second legs 202 and 204 of stent graft 36 because of these lower diameter regions may be more susceptible to occlusion than main body 206 of stent graft 36. Modified inner surface portion 200 may also include at least a portion of the inner surface of first branch limb extension 52 and/or second branch limb extension 54 to protect these relatively low diameter regions from occlusion. Any, all, or any sub-combination of the above portions may be modified.

A modified inner surface portion may be formed by treating an unmodified inner surface portion with a plasma material. In one or more embodiments, the modified inner surface portion is configured to decrease thrombogenicity of the inner surface portion to resist and prevent thrombus formation and/or limb graft occlusions. The modified inner surface portion may be configured to promote strong endothelial adhesion for better blood compatibility. The modified inner surface portion may have one or more inner surface portion characteristics (e.g., surface friction, surface smoothness, and/or hydrophilicity) to decrease thrombogenicity of the inner surface portion. A surface treatment may be applied that aligns with the flow direction through the lumen of the stent graft to promote endothelialization and to reduce thrombus formation. The surface treatment may be texturing, roughness, and or a hydrophilic characteristic. In one or more embodiments, the surface may be treated to be smooth (e.g., no or little roughness such as 0.1, 0.5, 1, 2, 3, or 4 nanometers) to reduce or prevent microcirculations and/or nanocirculations that can induce clot formation, thereby creating more hemocompatible material characteristics to reduce thrombus formation. The one or more surface portion characteristics may be imparted to the modified inner surface portion using one or more plasma treatment parameters (e.g., feed gas, power applied, treatment duration, and/or feed molecules). Non-limiting examples of feed gases/molecules include oxygen, argon, nitrogen, allylamine, aminopropyl silane, heparin, aminated-siloxanes, and combinations thereof. Nitrogen may be used for activation. Power, flux, and/or duration may be adapted to the treatment conditions (e.g., type of plasma and/or treatment region size). The plasma may be vacuum or atmospheric. Possible treatments include chemical vapor deposition (CVD) or plasma-enhanced chemical vapor deposition (PECVD).

The plasma treatment may form a mechanically modified inner surface portion to decrease thrombogenicity of the inner surface portion. The mechanically modified inner surface portion may have beneficial surface friction and/or surface smoothness to decrease thrombogenicity. The surface friction of the modified inner surface portion may be lower than the surface friction of the unmodified inner surface portion. The surface smoothness of the modified inner surface portion may be higher than the surface smoothness of the unmodified inner surface portion. The surface smoothness may be characterized as a deviation in surface roughness on a nanoscale and/or a microscale. The deviation may be any of the following percentages or in a range of any two of the following percentages: 0.1%, 0.2%, 0.3%, 0.4%, 0.5%, 0.6%, 0.7%, 0.8%, 0.9%, 1.0%, 2.0%, 3.0%, and 4.0%.

The plasma treatment may form a molecular modified inner surface portion to decrease thrombogenicity and/or promote strong endothelial adhesion. The molecular modified inner surface portion may include molecules that are covalently bonded to the inner surface portion. The plasma treatment may include treating the inner surface portion with molecules to form moieties on the inner surface portion to decrease thrombogenicity and/or promote strong endothelial adhesion. Non-limiting examples of feed molecules for this purpose include heparin, albumin, polyethylene glycol (PEG), antithrombin, and endothelial cell-derived molecules. Other examples include acrylates, siloxanes, and/or vinyl groups containing precursors with amino, hydroxyl, carboxyl and/or epoxy functionalities.

The plasma treatment may form a functional modified inner surface portion to decrease thrombogenicity and/or promote strong endothelial adhesion. For instance, the function of the inner surface may be modified to increase the hydrophilicity of the inner surface portion. For example, using an oxygen or argon feed gas to the plasma treatment may increase the hydrophilicity and/or the smoothness of the inner surface portion.

The modified inner surface portion may include one or more (e.g., any or all) of the modifications (e.g., mechanical, molecular, and functional modifications).

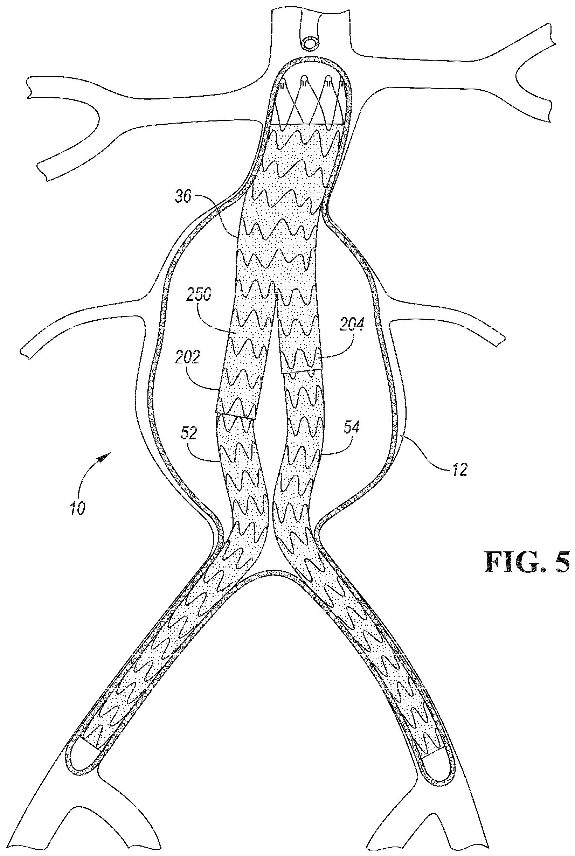

Figure 5 is a partial cross section view of abdominal aorta 10 with aneurysm 12 and a side view of stent graft 36, first branch limb extension 52, and second branch limb extension 54 shown in an expanded, deployed configuration and depicting modified outer surface portion 250. As shown in Figure 5, modified outer surface portion 250 includes all exposed outer surface portions of stent graft 36, first branch limb extension 52, and second branch limb extension 54. In other embodiments, modified outer surface portion 250 may only extend to selected portions of the exposed outer surfaces. For example, modified outer surface portion 250 may include at least a portion of the inner surface of stent graft 36. As another example, modified outer surface portion 250 may include at least a portion of the inner surface of first and second legs 202 and 204 of stent graft 36 because of these portions align the region of aneurysm 12 that benefits from modified outer surface portion 250. Modified outer surface portion 250 may also include at least a portion of the outer surface of first branch limb extension 52 and/or second branch limb extension 54. Modified outer surface portion 250 may include (or only include) a portion of the other surface aligning with an aneurysmal region spanning one or more aneurysms of one or more vessels. Any, all, or any sub-combination of the above portions may be modified.

A modified outer surface portion may be formed by treating an unmodified outer surface portion with a plasma material. In one or more embodiments, the modified outer surface portion is configured to increase thrombogenicity and/or to promote immune system modulation (e.g., immune cell modulation). The increased thrombogenicity may induce thrombosis of the sac of aneurysm 12 to resist Type 2 endoleaks. The immune system modulation may resist or prevent biofouling and/or graft infections. The immune cell modulation may steer foreign body response toward healing. The modified outer surface portion may have one or more outer surface portion characteristics (e.g., surface roughness and hydrophilicity) to increase thrombogenicity and/or to promote immune system modulation. In one or more embodiments, a smooth and/or hydrophilic (e.g., having a contact angle between 0° and 90°) surface induces a better healing response. The one or more surface portion characteristics may be imparted to the modified outer surface portion using one or more plasma treatment parameters (e.g., feed gas, power applied, treatment duration, and/or feed molecules).

The plasma treatment may form a mechanically modified outer surface portion with increased surface roughness to increase thrombogenicity using increased surface area for blood reactions to induce circulation and stagnation of the blood so that it can clot together. The mechanically modified outer surface portion may also promote wound healing (e.g., cellular reaction) and/or integration of the stent into the vessel. The surface roughness may be on a nanoscale or a microscale. The surface roughness of one or more embodiments may be any of the following values or in a range of any two of the following values: 5, 6, 7, 8, 9, 10, 11, 12, 13, 14, 15, 20, 25, 30, 35, 40, 45, and 50 nanometers to increase thrombogenicity. The plasma treatment may form a molecular modified outer surface portion to promote natural thrombus and/or to increase thrombogenicity. Non-limiting examples of feed molecules for this purpose include tissue factor molecules, fibrinogen, von Willebrand factor (vWF) molecules, and molecules with collagen moieties.

In another embodiment, the plasma treatment may form a molecular modified outer surface portion to increase hydrophilicity of the surface to reduce protein adsorption and biofouling. Non-limiting examples of feed molecules for this purpose include polydimethylsiloxane (PDMS) siloxanes, and fluorinated, long chain hydrocarbons. Plasmaactivated PDMS nanopillar arrays may enhance antifouling behavior (e.g., reduction in Escherichia coli adhesion). PEG-derivative molecules may be functionalized on the modified outer surface portion to create a non-fouling surface resistant to protein adsorption and cell adhesion and bacteria. In one or more embodiments, the modified outer surface portion may include an antimicrobial agent coating (e.g., citric acid, quaternary ammonium (e.g., virucidals and biocidals), and PEG-derivatives) to be ani-fouling.

In one or more embodiments, the modified inner surface portion may have one or more surface characteristics to steer foreign body response toward healing. The one or more surface characteristics may support adsorption of pro-healing cytokines (e.g., IL-10, IL4, and IL13), growth factors (e.g., PDGF, VEGF, and TGF), and/or tissue remodeling inhibitors (TIMPs).

Plasma treatment may also modify the stiffness of the implant material. The modifications may depend on the type of material of the implant and the specific plasma treatment parameters used. A thermal barrier coating may be treated with plasma to reduce macroscopic stiffness, which may enhance stability by limiting stresses from differential thermal contraction. Plasma treatment may also be used to functionalize the surface of the implant with immobilized antibodies, small proteins, peptides, aptamers, oligosaccharides, and enzymes.

While embodiments herein have been described with respect to a single type of plasma treatment (e.g., sealing in Fig. 2, anti-thrombogenic in Fig. 4, and thrombogenic in Fig. 5), a single stent graft may have any combination of any of the treatments disclosed herein. For example, a stent graft may have a plasma treatment to enhance outer surface sealing at the proximal end and/or at distal ends and also have a different plasma treatment on an interior surface to enhance endothelization or reduce thrombogenicity on an interior surface. In another example, a stent graft may have the above treatments and also a third plasma treatment on the outer surface of the stent graft within the aneurysm sac to increase thrombogenicity to reduce Type 2 endoleaks and promote sac regression. Other combinations are also contemplated herein.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

The following examples are illustrative of the techniques described herein.

Example 1. An endovascular implant comprising: a tubular body extending along a longitudinal axis and bounded by a proximal end and a distal end, the tubular body having an outer surface and an inner surface, the tubular body having an outer surface region including the outer surface and an inner surface region including the inner surface and a bulk region extending therebetween, the tubular body includes an outer surface landing region within the outer surface region and proximate at least one of the proximal and distal ends of the tubular body, the outer surface landing region is configured to align with one or more landing walls of one or more vessels, the outer surface landing region includes a plasma modified outer surface landing region having a different outer surface compared to an unmodified outer surface landing region.

Example 2. The endovascular implant of Example 1, wherein the plasma modified outer surface landing region is configured to promote cellular adhesion and/or cellular ingrowth.

Example 3. The endovascular implant of Example 1, wherein the plasma modified outer surface landing region has one or more modified characteristics and the unmodified outer surface landing region has one or more unmodified characteristics.

Example 4. The endovascular implant of Example 3, wherein the one or more modified characteristics includes a modified surface roughness and the one or more unmodified characteristics includes an unmodified surface roughness, and the modified surface roughness is greater than the unmodified surface roughness.

Example 5. The endovascular implant of Example 1, wherein the plasma modified outer surface landing region includes covalently bonded molecules imparted from feed molecules of a plasma treatment.

Example 6. The endovascular implant of Example 5, wherein the feed molecules are one or more amine molecules, one or more molecules having hydroxyl groups, one or more molecules having carboxyl groups, or a combination thereof.

Example 7. The endovascular implant of Example 5, wherein the feed molecules are one or more biologic molecules, the biologic molecules are selected from the group consisting of collagen, hyaluronic acid, fibronectin, amine molecules, microRNA, antimicrobial peptides, or a combination thereof.

Example 8. The endovascular implant of Example 1, wherein the plasma modified outer surface landing region includes alternating first and second modified regions formed using first and second plasma treatments, the second plasma treatment is different than the first plasma treatment, the first modified regions have a first surface roughness, the second modified surface region has a second surface roughness, and the first surface roughness is different than the second surface roughness.

Example 9. An endovascular implant comprising: a tubular body extending along a longitudinal axis and bounded by a proximal end and a distal end, the tubular body having an outer surface and an inner surface, the tubular body having an outer surface region including the outer surface and an inner surface region including the inner surface and a bulk region extending therebetween, the inner surface region is configured to contact blood in a deployed state of the endovascular implant, the inner surface region includes a plasma modified inner surface region having a different inner surface compared to an unmodified inner surface region.

Example 10. The endovascular implant of Example 9, wherein the plasma modified inner surface region is configured to decrease thrombogenicity from the blood.

Example 11. The endovascular implant of Example 9, wherein the plasma modified inner surface region has one or more modified characteristics and the unmodified inner surface region has one or more unmodified characteristics.

Example 12. The endovascular implant of Example 10, wherein the one or more modified characteristics includes a modified surface smoothness and the one or more unmodified characteristics includes an unmodified surface smoothness, and the modified surface smoothness is greater than the unmodified surface smoothness.

Example 13. The endovascular implant of Example 9, wherein the plasma modified inner surface region includes covalently bonded molecules imparted from feed molecules of a plasma treatment.

Example 14. The endovascular implant of Example 12, wherein the feed molecules are heparin, albumin, polyethylene glycol (PEG), antithrombin, endothelial cell-derived molecules, and/or combinations thereof.

Example 15. The endovascular implant of Example 12, wherein the feed molecules are acrylates, siloxanes, and/or vinyl groups containing precursors with amino, hydroxyl, carboxyl and/or epoxy functionalities.

Example 16. An endovascular implant comprising: a tubular body extending along a longitudinal axis and bounded by a proximal end and a distal end, the tubular body having an outer surface and an inner surface, the tubular body having an outer surface region including the outer surface and an inner surface region including the inner surface and a bulk region extending therebetween, the outer surface region includes a plasma modified outer surface region having a different outer surface compared to an unmodified outer surface region.

Example 17. The endovascular implant of Example 15, wherein the plasma modified outer surface region is configured to increase thrombogenicity and/or to promote immune system modulation.

Example 18. The endovascular implant of Example 15, wherein the plasma modified outer surface region has one or more modified characteristics and the unmodified outer surface region has one or more unmodified characteristics.

Example 19. The endovascular implant of Example 17, wherein the one or more modified characteristics includes a modified surface roughness and the one or more unmodified characteristics includes an unmodified surface roughness, and the modified surface roughness is greater than the unmodified surface roughness.

Example 20. The endovascular implant of Example 15, wherein the plasma modified outer surface landing region includes covalently bonded molecules imparted from feed molecules of the plasma treatment, and the feed molecules include one or more polydimethylsiloxane (PDMS), siloxanes, fluorinated, long chain hydrocarbons, or a combination thereof.

## Claims

1. An endovascular implant (36) comprising:
a tubular body (42) extending along a longitudinal axis and bounded by a proximal end (44) and a distal end (46), the tubular body having an outer surface and an inner surface, the tubular body having an outer surface region including the outer surface and an inner surface region including the inner surface and a bulk region extending therebetween, the inner surface region and/or the outer surface region include a plasma modified inner surface region (200) having a different inner surface compared to an unmodified inner surface region and/or a plasma modified outer surface region (100, 150 or 250) having a different outer surface compared to an unmodified outer surface region.

2. The endovascular implant of claim 1, wherein the tubular body includes an outer surface landing region (38) within the outer surface region and proximate at least one of the proximal and distal ends of the tubular body, the outer surface landing region is configured to align with one or more landing walls of one or more vessels, the outer surface landing region includes a plasma modified outer surface landing region (100) having a different outer surface compared to an unmodified outer surface landing region.

3. The endovascular implant of claim 2, wherein the plasma modified outer surface landing region is configured to promote cellular adhesion and/or cellular ingrowth.

4. The endovascular implant of claim 2, wherein the plasma modified outer surface landing region has one or more modified characteristics and the unmodified outer surface landing region has one or more unmodified characteristics, the one or more modified characteristics includes a modified surface roughness and the one or more unmodified characteristics includes an unmodified surface roughness, and the modified surface roughness is greater than the unmodified surface roughness.

5. The endovascular implant of claim 2, wherein the plasma modified outer surface landing region includes covalently bonded molecules imparted from feed molecules of a plasma treatment.

6. The endovascular implant of claim 5, wherein the feed molecules are one or more amine molecules, one or more molecules having hydroxyl groups, one or more molecules having carboxyl groups, or a combination thereof.

7. The endovascular implant of claim 5, wherein the feed molecules are one or more biologic molecules, the biologic molecules are selected from the group consisting of collagen, hyaluronic acid, fibronectin, amine molecules, microRNA, antimicrobial peptides, or a combination thereof.

8. The endovascular implant of claim 2, wherein the plasma modified outer surface landing region includes alternating first and second modified regions (152 and 154) formed using first and second plasma treatments, the second plasma treatment is different than the first plasma treatment, the first modified regions have a first surface roughness, the second modified surface region has a second surface roughness, and the first surface roughness is different than the second surface roughness.

9. The endovascular implant of claim 1, wherein the inner surface region and/or the outer surface region is the plasma modified inner surface region, the plasma modified inner surface region is configured to decrease thrombogenicity from the blood.

10. The endovascular implant of claim 9, wherein the plasma modified inner surface region has one or more modified characteristics and the unmodified inner surface region has one or more unmodified characteristics, the one or more modified characteristics includes a modified surface smoothness and the one or more unmodified characteristics includes an unmodified surface smoothness, and the modified surface smoothness is greater than the unmodified surface smoothness.

11. The endovascular implant of claim 9, wherein the plasma modified inner surface region includes covalently bonded molecules imparted from feed molecules of a plasma treatment.

12. The endovascular implant of claim 11, wherein the feed molecules are heparin, albumin, polyethylene glycol (PEG), antithrombin, endothelial cell-derived molecules, and/or combinations thereof.

13. The endovascular implant of claim 11, wherein the feed molecules are acrylates, siloxanes, and/or vinyl groups containing precursors with amino, hydroxyl, carboxyl and/or epoxy functionalities.

14. The endovascular implant of claim 1, wherein the inner surface region and/or the outer surface region is the plasma modified outer surface region, the plasma modified outer surface region is configured to increase thrombogenicity and/or to promote immune system modulation.

15. The endovascular implant of claim 14, wherein the plasma modified outer surface landing region includes covalently bonded molecules imparted from feed molecules of the plasma treatment, and the feed molecules include one or more polydimethylsiloxane (PDMS), siloxanes, fluorinated, long chain hydrocarbons, or a combination thereof.
